# EUROPEAN PATENT APPLICATION

(11) **EP 1 308 729 A2**
(43) Date of publication of application: **07.05.2003**
(21) Application number: 02023743.4
(22) Date of filing: 24.10.2002
(51) Int. Cl.: G01N 33/569

(54) **Serological assay for neospora caninum**

(30) Priority: 31.10.2001 US 334811 P
(71) Applicant: Pfizer Products Inc., Groton, Connecticut 06340 (US)
(72) Inventor: Brake, David A., East Lyme, Connecticut (US); Ritter, Dianne M., East Greenwich, Rhode Island (US)
(74) Representative: Tesch, Rudolf

(57) **Abstract**

The present invention relates to the serological detection of *Neospora caninum*-vaccinated animals by means of reaction of a subject serum with a protein reactive with *N*. *caninum.* The protein may be a full-length native or recombinant *N. caninum* or *Toxoplasma gondii* bradyzoite fusion protein or a truncated fusion protein or fragment thereof. The protein may be used in any of a number of assays including enzyme linked immunosorbant assays (ELISA), a radioimmunoassay (RIA), a Western Blot and other suitable forms of immunoassay.

SEQUENCE LISTING

<110> PFIZER PRODUCTS INC.

<120> SEROLOGICAL ASSAY FOR NEOSPORA CANINUM

<130> PC23020A

<140>
<141>

<160> 4

<170> PatentIn Ver. 2.1

<210> 1
<211> 1219
<212> DNA
<213> Toxoplasma gondii

<220>
<221> CDS
<222> (219)..(905)

<220>
<221> CDS
<222> (303)..(905)

<400> 1
tgcttttgcc aaaggagacc tgtgtgcaga ggacttcgct gctaaaaagc agaagagtgc 60

acggcgtgtg tagctcagtg gcatttcggg actcggtctt gcgtcgttcg cgactggacg 120

tcgtcgtctg tgagagcgtc aaactaggga gaaggggcgg gccagagcgt tcggaaaatt 180

atctgcaaag cccaggtccc gtatgatatt caaaaaag atg gcg ccg tca gca tcg 236
                                         Met Ala Pro Ser Ala Ser
                                           1               5

cat ccg ccg gga gct tgt cca ccg gga tgt acc aag cat cct gct act    284
His Pro Pro Gly Ala Cys Pro Pro Gly Cys Thr Lys His Pro Ala Thr
            10                  15                  20

gcg aca gcc att tcc ccg agt gga gtg tgt ccc atg cgt gcg ttc cat    332
Ala Thr Ala Ile Ser Pro Ser Gly Val Cys Pro Met Arg Ala Phe His
        25                  30                  35

ccc gcg ggg cct cac tca cat ttc tca tgt tac gat gat ctc aga aat    380

1

Pro Ala Gly Pro His Ser His Phe Ser Cys Tyr Asp Asp Leu Arg Asn
    40                  45                  50

agg ctg agt cac gac aaa aat gtt cga ccg gtc gcc tct caa cag cta 428
Arg Leu Ser His Asp Lys Asn Val Arg Pro Val Ala Ser Gln Gln Leu
55                  60                  65                  70

gac tat ttg gat gaa gta agc ccg ttt gct ctg gcg tac tac cct ccc 476
Asp Tyr Leu Asp Glu Val Ser Pro Phe Ala Leu Ala Tyr Tyr Pro Pro
                75                  80                  85

ccg ttt tgg ggt gga gtc ggt ctt aat ccc atc gac gat atg ttg ttc 524
Pro Phe Trp Gly Gly Val Gly Leu Asn Pro Ile Asp Asp Met Leu Phe
            90                  95                  100

gag acg gcc ctc acc gca aac gaa atg atg gag gac atc acg tgg aga 572
Glu Thr Ala Leu Thr Ala Asn Glu Met Met Glu Asp Ile Thr Trp Arg
        105                 110                 115

ccc aga gtc gac gtg gag ttc gac agc aaa aag aag gaa atg att att 620
Pro Arg Val Asp Val Glu Phe Asp Ser Lys Lys Lys Glu Met Ile Ile
    120                 125                 130

ttg gct gac ttg cca ggt ctt cag aaa gat gac gta acc ata gaa gtc 668
Leu Ala Asp Leu Pro Gly Leu Gln Lys Asp Asp Val Thr Ile Glu Val
135                 140                 145                 150

gac aac gga gcc atc gtt atc aaa gga gag aag acc tcg aaa gaa gcg 716
Asp Asn Gly Ala Ile Val Ile Lys Gly Glu Lys Thr Ser Lys Glu Ala
                155                 160                 165

gag aaa gtg gac gat ggc aaa aca aag aac att ttg act gag cga gtg 764
Glu Lys Val Asp Asp Gly Lys Thr Lys Asn Ile Leu Thr Glu Arg Val
            170                 175                 180

tcc ggt tat ttt cgc gcc cgg ttc cag ctc ccg agt aat tac aag ccc 812
Ser Gly Tyr Phe Arg Ala Arg Phe Gln Leu Pro Ser Asn Tyr Lys Pro
        185                 190                 195

gac gga atc agt gcg gca atg gac aac ggc gtt cta cgt gtc acg atc 860
Asp Gly Ile Ser Ala Ala Met Asp Asn Gly Val Leu Arg Val Thr Ile
    200                 205                 210

aag gtc gag gat tca ggg ggc gca aag caa caa atc agc gtg aag 905
Lys Val Glu Asp Ser Gly Gly Ala Lys Gln Gln Ile Ser Val Lys
215                 220                 225

tagaggcagc gatgccgttc gtggggcagg gaaacacgga ggagcctcat 955

2

t gaaaatgtaa aggtgtggga gaactgttga cagtgcaaga taatagtacg 1006

agtaggattg caaagaaggc ctcccgtttc tggcgccagc cgagaaaacc tggttgatgg 1066

gcagcctcat gcgtgacggt tttctttag aggacttgtc cgtcgaggta gtgcgtatcg 1126

cgtgtctgcg gtttatcgtc acagcgcgta tccagaaacc tagcgttcaa cggctcaaga 1186

ccgtaagcga attagagttg aataggtgtg ttat 1220

<210> 2
<211> 229
<212> PRT
<213> Toxoplasma gondii

<400> 2
Met Ala Pro Ser Ala Ser His Pro Pro Gly Ala Cys Pro Pro Gly Cys
1               5                   10                  15
Thr Lys His Pro Ala Thr Ala Thr Ala Ile Ser Pro Ser Gly Val Cys
            20                  25                  30
Pro Met Arg Ala Phe His Pro Ala Gly Pro His Ser His Phe Ser Cys
        35                  40                  45
Tyr Asp Asp Leu Arg Asn Arg Leu Ser His Asp Lys Asn Val Arg Pro
    50                  55                  60
Val Ala Ser Gln Gln Leu Asp Tyr Leu Asp Glu Val Ser Pro Phe Ala
65                  70                  75                  80
Leu Ala Tyr Tyr Pro Pro Pro Phe Trp Gly Gly Val Gly Leu Asn Pro
                85                  90                  95
Ile Asp Asp Met Leu Phe Glu Thr Ala Leu Thr Ala Asn Glu Met Met
            100                 105                 110
Glu Asp Ile Thr Trp Arg Pro Arg Val Asp Val Glu Phe Asp Ser Lys
        115                 120                 125
Lys Lys Glu Met Ile Ile Leu Ala Asp Leu Pro Gly Leu Gln Lys Asp
    130                 135                 140
Asp Val Thr Ile Glu Val Asp Asn Gly Ala Ile Val Ile Lys Gly Glu
145                 150                 155                 160
Lys Thr Ser Lys Glu Ala Glu Lys Val Asp Asp Gly Lys Thr Lys Asn
                165                 170                 175
Ile Leu Thr Glu Arg Val Ser Gly Tyr Phe Arg Ala Arg Phe Gln Leu
            180                 185                 190
Pro Ser Asn Tyr Lys Pro Asp Gly Ile Ser Ala Ala Met Asp Asn Gly
        195                 200                 205
Val Leu Arg Val Thr Ile Lys Val Glu Asp Ser Gly Gly Ala Lys Gln
    210                 215                 220
Gln Ile Ser Val Lys
225

3

<210> 3
<211> 201
<212> PRT
<213> Toxoplasma gondii

<400> 3
Ser Gly Val Cys Pro Met Arg Ala Phe His Pro Ala Gly Pro His Ser
1               5                   10                  15
His Phe Ser Cys Tyr Asp Asp Leu Arg Asn Arg Leu Ser His Asp Lys
            20                  25                  30
Asn Val Arg Pro Val Ala Ser Gln Gln Leu Asp Tyr Leu Asp Glu Val
        35                  40                  45
Ser Pro Phe Ala Leu Ala Tyr Tyr Pro Pro Pro Phe Trp Gly Gly Val
    50                  55                  60
Gly Leu Asn Pro Ile Asp Asp Met Leu Phe Glu Thr Ala Leu Thr Ala
65                  70                  75                  80
Asn Glu Met Met Glu Asp Ile Thr Trp Arg Pro Arg Val Asp Val Glu
                85                  90                  95
Phe Asp Ser Lys Lys Lys Glu Met Ile Ile Leu Ala Asp Leu Pro Gly
            100                 105                 110
Leu Gln Lys Asp Asp Val Thr Ile Glu Val Asp Asn Gly Ala Ile Val
        115                 120                 125
Ile Lys Gly Glu Lys Thr Ser Lys Glu Ala Glu Lys Val Asp Asp Gly
    130                 135                 140
Lys Thr Lys Asn Ile Leu Thr Glu Arg Val Ser Gly Tyr Phe Arg Ala
145                 150                 155                 160
Arg Phe Gln Leu Pro Ser Asn Tyr Lys Pro Asp Gly Ile Ser Ala Ala
                165                 170                 175
Met Asp Asn Gly Val Leu Arg Val Thr Ile Lys Val Glu Asp Ser Gly
            180                 185                 190
Gly Ala Lys Gln Gln Ile Ser Val Lys
        195                 200

<210> 4
<211> 672
<212> DNA
<213> Neospora caninum

<400> 4
cccctttctt ctctccgttt caagatgagc aagctcagca cagacggcct gaagaaggcc 60
atcggagaga tcttggaggg ctcgcgggaa aagaagagaa agttcgtgga gaccgtcgag 120
cttcagatcg gtttgaagga ttacgacacc caaagagaca agcgtttcag cggaagcgtg 180
aggcttccta acgtcccccg cccccgcatg cgcgtgtgcg tgatgggaga tgctgtgcat 240
tgcgagcaag caaaggagct gggactggaa ttcatggacg tggaggctat gaagaagttg 300

4

aacaagaaca agaagcttgt gaagaaactc gcacggaagt acgacgcttt cctggcctcg 360
caagtgttga ttccgcagat cccccgtctc ctgggtccgg gtcttaacaa ggcgggcaaa 420
ttcccgacgc ttatcactca caacgataag ctcgaggata agattcagga natcaagagc 480
tcaatcaaat tccaactcag aaaggtgcct gtgcatggtg tcgccgtcng gcacgtcgac 540
atgacggagg agcaactgcg cgtgaacctg acactggcca tcaacttcct tgtctctctg 600
ctgaagaaga actggaacag cgtgagaacg ctgcacatca agagcaccat gggcaagcct 660
cagcagattt ac 672

5

## Description

### FIELD OF THE INVENTION

The present invention relates to the serological detection of *Neospora caninum* infection by means of reaction of a subject serum with a protein reactive with *N. caninum.*

### BACKGROUND OF THE INVENTION

*Neospora caninum* and *Toxoplasma gondii* are closely related protozoan parasites responsible for disease in a wide number of animals. *N. caninum* has been recognized as a cause of abortion, neurologically-associated limb defects and neonatal mortality in cattle and other animals. *T. gondii* is a common cause of ovine abortion. Effective management of neosporosis and toxoplasmosis requires rapid diagnosis. The development of efficient diagnostic tests for *N*. *caninum* is therefore critical.

There are several art-recognized tests available to determine whether or not an animal has been exposed to *N.caninum.* However, diagnostic tests and methods for distinguishing between *N. caninum* naturally seropositive animals and *N. caninum* vaccinated and/or *N. caninum* seronegative (unvaccinated) animals have heretofore not been developed.

### SUMMARY OF THE INVENTION

The present invention, for the first time, provides a method for distinguishing a *N. caninum* naturally seropositive animal from an animal vaccinated with a Neospora vaccine. The present invention also provides a method for determining whether or not an animal has been infected with *N*. *caninum* using a serological assay.

One aspect of the present invention provides a method for detecting antibodies to *Neospora caninum* in a serum sample by contacting the serum with a *Toxoplasma gondii* protein and examining the sera for the presence of bound antibody.

### BRIEF DESCRIPTION OF THE FIGURES

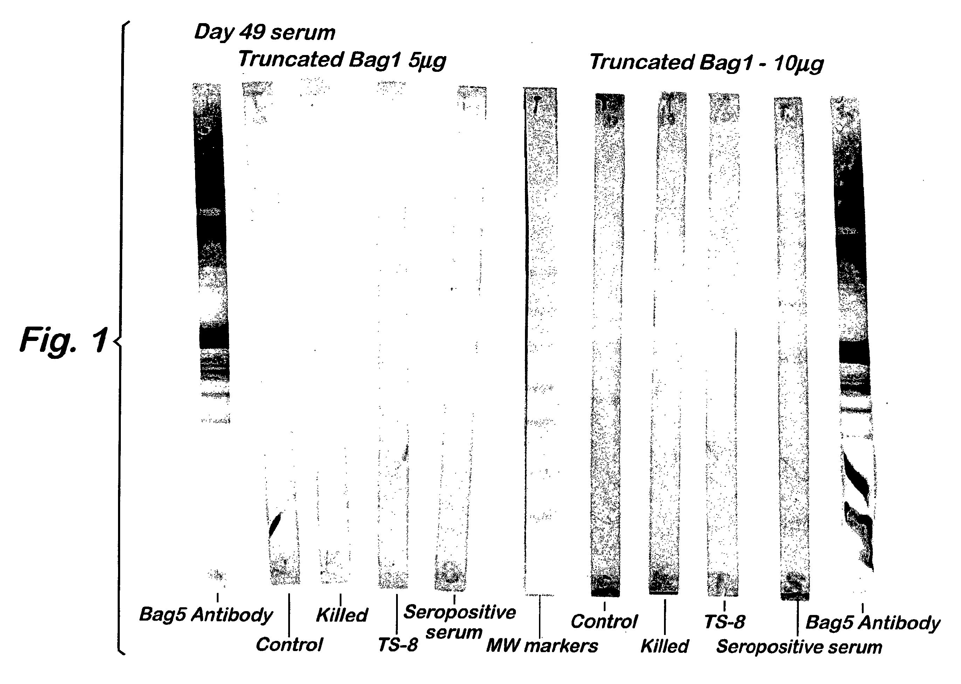

Figure 1 shows Western blot reactivity against truncated BAG1-GST
   - Lane 1:: rabbit antisera raised against full-length BAG1-GST (positive control)
   - Lane 2:: serum from naive, seronegative cow (negative control)
   - Lane 3:: day 49 pooled antisera from *N. caninum* killed vaccine immunized cows
   - Lane 4:: day 49 pooled antisera from *N. caninum* attenuated vaccine immunized cows
   - Lane 5:: pooled antisera from naturally infected seropositive cows
   - Lane 6:: MW markers (kDa)

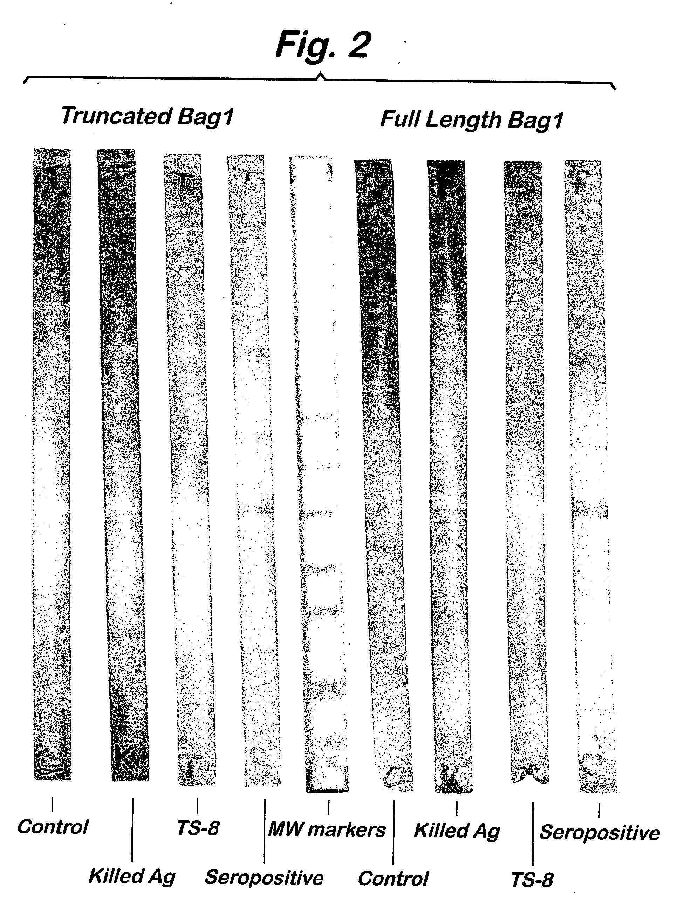

Figure 2 shows Western blot reactivity against truncated and full-length BAG1-GST
   - Lanes 1-4:: truncated BAG1-GST antigen
   - Lane 5:: MW markers (kDa)
   - Lanes 6-9:: full-length BAG1-GST antigen
   - Lanes 1,6:: serum from naïve, seronegative cow (negative control)
   - Lanes 2, 7:: day 119 pooled antisera from *N. caninum* killed vaccine immunized cows
   - Lanes 3, 8:: day 119 pooled antisera from *N. caninum* attenuated vaccine immunized cows
   - Lanes 4, 9:: pooled antisera from naturally infected seropositive cows
   - Lane 5:: MW markers (kDa).

### DETAILED DESCRIPTION OF THE INVENTION

The present invention provides an assay system which is able to distinguish between vaccinated and naturally exposed Neospora-seropositive animals. In accordance with the present invention, proteins from *Toxoplasma gondii* and *N. caninum* have been found to be present only in naturally seropositive animals. *N. caninum* seronegative animals and animals which have been previously vaccinated with Neospora-based vaccines (see, e,g. U.S. Serial No. 09/260,414 "Attenuated Live Neospora Vaccine", incorporated herein by reference and U.S. Serial No. 09/138,985 "Neospora Vaccine", incorporated herein by reference) can now be reliably identified and sequestered from seropositive (i.e. naturally exposed) animals based on the assay results provided in accordance with the methods of the present invention.

Sera from animals exposed to *N.caninum* will react with *Toxoplasma gondii* and *N. caninum* proteins and fragments thereof. By "react" is meant a detectable antigen-antibody complex will be formed. The formation of an antigen-antibody complex is indicative of an animal naturally infected with *N. caninum.* Sera from animals previously vaccinated with *N. caninum* and sera from animals naturally seronegative for *N. caninum* will not react with *Toxoplasma gondii* and *N. caninum* proteins and fragments thereof. Accordingly, the invention provides a method to distinguish between vaccinated (seronegative) and naturally exposed *Neospora caninum*-seropositive animals

Moreover, in accordance with the present invention, *N. caninum* seropositive animals can be vaccinated based on the results of the assay of the present invention, if desired. Animals contemplated by the present invention include cattle, calves, bulls, cows and steer. Preferably, the method of the present invention is applied to cows and most preferably to a calf.

In accordance with the present invention, any purified, native or recombinant bradyzoite antigen or fragment thereof from *Toxoplasma gondii* or *Neospora caninum,* can be employed as a detection antigen for the serological assay of the invention. By "fragment" is meant a peptidic portion of the full-length *T.* gondii or *N caninum* protein which provides an epitope which is recognized by antibodies present in animal sera. Truncated forms of the full-length *T.* gondii and *N caninum* proteins are also understood to be fragments, in accordance with the present invention.

In one embodiment, full-length bradyzoite antigen glutathione-S-transfersase (GST) fusion protein (BAG-1) is the detection antigen, (SEQ ID NO. 2). In another embodiment, a truncated BAG-1-GST fusion protein, lacking the N-terminal 28 amino acids of BAG1 is the detection antigen, (SEQ ID NO. 3). BAG-1 is also known in the art as BAG-5. The present invention contemplates that antisera from an animal naturally infected with *N. caninum* or antisera from an animal vaccinated with *N. caninum* or uninfected antisera can be employed as the test sample.

The reactivity of a bradyzoite antigen or fragment thereof when immobilized on a solid support provides a method of detecting *N*. *caninum* antibodies in accordance with the present invention. Specifically, the methods of the invention can be conducted by contacting a sample of animal serum with the bradyzoite-stage protein or fragment thereof, which can then react to form an antigen-antibody complex, and examining the resulting complex for reaction by a conventional detection and quantitation methods. By "antibody" is meant an immunoglobulin molecule able to bind to a specific epitope on an antigen. Antibodies can be a polyclonal mixture or a monoclonal. Antibodies can be intact immunoglobulins derived from natural sources or from recombinant sources and can be immunoreactive portions of intact immunoglobulins.

Numerous methods of examining the quantity and/or the presence or absence of the antigen-antibody complex are known and all are contemplated by the present invention. For example, a Western blot assay may be performed by attaching the antigen to a solid support, such as nitrocellulose paper, incubating the paper with a serum test sample, and examining the nitrocellulose paper for the presence or absence of a specific band at the expected molecular weight for the full length (about 55 kDa) or truncated (about 51 kDa) *T. gondii* BAG1-GST protein. The presence of a band at either of the expected molecular weights indicates that the animal is seropositive for *N. caninum.* Conversely, the absence of a band at either of the expected molecular weights indicates that the animal is vaccinated.

The present invention also contemplates a Western blot assay wherein the antigen is attached to a nitrocellulose paper and the antigen is stained with an antibody which has a dye attached. An enzyme-linked immunosorbant assay (ELISA) can also be employed where the antigen or antibody is labeled with an enzyme. The present invention also contemplates a radioimmunoassay wherein the antigen or antibody is labeled with a radioactive element.

The present invention contemplates a method of examining animal serum for the presence/quantity or absence of *N. caninum* antibodies. Thus, a diagnostic assay kit containing the assay and controls for positive and/or negative reactions, with other necessary ingredients can be assembled in accordance with the teachings of the present invention.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

### EXAMPLE 1

### Antisera from N. caninum seronegative, N. caninum vaccinated animals

Any serum sample from a seronegative or PCR negative animal that subsequently receives a *N.caninum* tachyzoite based modified-live, killed or subunit-based vaccine can be used. In this example, four-month old calves were determined to be seronegative by a diagnostic ELISA test conducted by a qualified veterinary diagnostic lab (Washington State Diagnostic Lab, Pullman, WA). Calves were vaccinated on day 0 and day 21 with either a tachyzoite-based i) modified-live, attenuated Neospora vaccine (USPTO patent application serial 09/260,414 "Attenuated Live Neospora Vaccine", incorporated herein by reference) or ii) inactivated, whole cell homogenate Neospora vaccine (USPTO patent application serial 09/138,985 "Neospora Vaccine", incorporated herein by reference). Both vaccines are based on tachyzoite antigens as the protective component of the vaccine. The *N. caninum* modified-live, attenuated vaccine contained 5 x 10⁷ Ncts-8 tachyzoites per dose and was administered subcutaneously to 3 seronegative animals. The *N. caninum* inactivated homogenate vaccine contained 100 ug total tachyzoite protein/dose, formulated in a saponin-based adjuvant (750 ug Quil A/150 ug cholesterol) and was administered subcutaneously to 3 seronegative animals. On days 49 and 119 post-vaccination, serum from the three calves in each vaccine group were collected, pooled and frozen in aliquots at -20°C until testing in the serological assay (see Example 4).

### EXAMPLE 2

### Antisera from N. caninum naturally infected, seropositive animals

Any serum sample from a naturally infected, seropositive or PCR positive animal can be used. In this example, the serological status of individual animals from a commercial cattle herd was determined using a diagnostic ELISA test conducted by a qualified veterinary diagnostic lab (Washington State Diagnostic Lab, Pullman, WA). Twenty-one animals reported as seropositive by this test were identified. A serum sample from each seropositive animal was collected, samples pooled, and frozen in aliquots at -20oC until testing in the serological assay (example 4).

### EXAMPLE 3:

### Bradyzoite Antigen Used in Neospora Serological Test

Any purified, native or recombinant *Neospora caninum* or *Toxoplasma gondii* bradyzoite protein can be used (*T. gondii* bradyzoite protein BAG1; Genebank Accession No: U23944). In this example, two different recombinant produced forms of the *T. gondii* bradyzoite antigen, BAG1 (also known in the art as BAGS) were used. The first form was a full-length BAG1-glutathione-S-transferase (GST) fusion protein expressed and purified from *E. coli* using affinity chromatograph (S. F. Parmley, et. al. 1995. Mol. Biochem. Parasit. 73: 253-257, incorporated herein by reference). The second form was a truncated BAG1-GST fusion protein, lacking the first 28 amino acids, expressed and purified from *E. coli* using affinity chromatography (Parmley et al., 1995). Purified, recombinant full-length BAG1-GST and truncated BAG1-GST proteins were stored frozen at -20°C until testing in the serological assay (example 4).

### EXAMPLE 4

### Serological Test

Any antibody- based detection test (ELISA, competitive ELISA, RIA, Western blot, etc.,) known in the art can be used. In this example, a Western blot assay was employed. A total of 5 mg *T. gondii* full-length BAG1-GST or truncated BAG1-GST recombinant protein was thawed, mixed with 5X denaturing sample loading buffer (Pierce, IL.) and loaded onto a 1mm x 2 well, precast 4-20% Tris-glycine continuous gradient gel (Novex, San Diego, CA). The gel was run according to manufacturer's specifications at 125V for 1.5 hrs. The gel was blotted onto a 20 uM nitrocellulose sheet (Bio-Read, Hercules, CA) at 25V for 1 hr. The sheet was dried and cut into equal length/width strips. The strips were incubated overnight at room temperature in a blocking solution (5% skim milk in phosphate-buffered saline (PBS)), rinsed in PBS and then incubated with a serum test sample (from example 1 or 2 above).

Aliquots of serum test samples from example 1 and 2 were thawed at room temperature and a freshly prepared 1:200 dilution in PBS was made. Serum from a naive, seronegative cow was used a negative control (1:200 dilution).Rabbit antisera against full-length BAG1-GST was used as a positive control (received as gift from M. McAllister; M. McAllister, et. al., 1996. J. Parasitol. 82(2): 354-355.) and was diluted 1:12,500 in PBS prior to use.

An individual test strip (prepared in example 3) was added to each diluted serum sample and incubated for 1 hr. at room temperature. The strips were briefly rinsed two times in PBS containing 0.05% Tween 20 (PBST) and added to a solution containing a 1:400 dilution (in PBS) of affinity purified phosphatase labeled goat anti-bovine IgG (KPL, Gaithersburg, MD.). Following a 1-hr incubation at room temperature, the strips were briefly rinsed two times in PBST and then incubated in a phosphatase substrate, BCIP/NBT (KPL, Gaithersburg, MD.). After the appropriate development of color (approximately 10 min) the enzymatic reaction was terminated by briefly placing the strips in distilled water. The strips were then air-dried. Results are shown in Figure 1.

### EXAMPLE 5:

### Interpretation of Serological Test

A positive serological test is indicated by the presence of a specific reaction between the serum test sample and the *N. caninum* or *T. gondii* bradyzoite antigen. In this example, the strips used in Example 4 were examined for a specific reaction by determining the presence or absence or a specific band at the expected molecular weight for the full-length (approximately 55 kDa) or truncated (51 kDa) *T. gondii* BAG1-GST protein.

As shown in Figure 1, lane 5, an approximately 52-55 kDa band was present in the strip incubated with the pooled sera from *N. caninum* naturally, infected cattle, indicating a specific reaction between the *N*. *caninum* naturally, infected seropositive test sample and the truncated BAG1-GST bradyzoite antigen. In contrast, in lanes 3 and 4 of Figure 1, no reactivity against the 55kDa BAG1-GST protein was detected, indicating the lack of a specific reaction using either of the *N. caninum* vaccinated test samples.

As shown in Figure 2, lanes 4 and 9, an approximately 52-55 kDa band was present in the strips incubated with the pooled sera from *N. caninum* naturally, infected cattle, indicating a specific reaction between the *N. caninum* naturally, infected seropositive test sample and the truncated (lane 4) or full-length (lane 9) BAG1-GST bradyzoite antigen. In contrast, in lanes 2 and 7 of Figure 2, no reactivity against either form of the BAG1-GST protein was detected using day 119 pooled antisera from cows immunized with the N. *caninum* killed vaccine. Similarly, in lanes 3 and 8 of Figure 2, no reactivity against either form of the BAG1-GST protein wad detected using day 119 pooled antisera from cows immunized with the *N. caninum* attenuated vaccine.

The difference in reactivity to the *T. gondii* recombinant bradyzoite antigen described in this invention demonstrates that a serological test using a bradyzoite antigen can be used to discriminate or diagnose a *N. caninum* naturally infected versus *N. caninum* vaccinated animal. Any purified, native or recombinant bradyzoite antigen or fragment thereof, from *N. caninum* (e.g. SEQ ID No. 1 or 2) or *N. caninum* (e.g. SEQ ID No. 3or 4) can be used as the detection antigen for this diagnostic test. Any antisera from *N. caninum* naturally infected or *N. caninum* vaccinated animals can be used as the test sample for this diagnostic test.

## Claims

1. A method for distinguishing an animal naturally seropositive for *N. caninum* from an animal vaccinated with a Neospora vaccine comprising obtaining a serum sample from the animal and contacting the serum with a Toxoplasma gondii protein and examining the serum for the presence of bound antibody.

2. The method of claim 1 wherein said *Toxoplasma gondii* protein comprises the amino acid sequence of SEQ ID NO:2.

3. The method of claim 1 wherein said *Toxoplasma gondii* protein comprises the amino acid sequence of SEQ ID NO:3.

4. The method of claim 2 wherein said *Toxoplasma gondii* protein is BAG-1.

5. The method of claim 2 wherein said *Toxoplasma gondii* protein is BAG-5

6. The method of claim 1 wherein the protein is attached to a support.

7. The method of claim 1 wherein the step of examining the protein for the presence of bound antibody is conducted by a Western Blot assay.

8. The method of claim 1 wherein the step of examining the protein for the presence of bound antibody is conducted by a radioimmunoassay.

9. The method of claim 1 wherein the step of examining the protein for the presence of bound antibody is conducted by an enzyme-linked immunosorbent assay.

10. The method of claim 1 further comprising quantifying the amount of antibody bound to the protein.

11. A. method for diagnosing an animal suspected of having been infected with *N. caninum* comprising obtaining a serum sample from the animal and contacting the serum with a *Toxoplasma gondii* protein and examining the serum for the presence of bound antibody.

12. The method of claim 11 wherein said *Toxoplasma gondii* protein comprises the amino acid sequence of SEQ ID NO:2 or the amino acid sequence of SEQ ID NO:3.

13. The method of claim 12 wherein said *Toxoplasma gondii* protein is BAG-1 or BAG-5.

14. A method for detecting antibodies to *Neospora caninum* in a serum sample comprising contacting the serum with a *Toxoplasma gondii* protein and examining the serum for the presence of bound antibody.

15. A method for distinguishing an animal naturally seropositive for *N*. *caninum* from an animal vaccinated with a Neospora vaccine comprising obtaining a serum sample from the animal and contacting the serum with a Toxoplasma gondii protein fragment and examining the serum for the presence of bound antibody.
